# EUROPEAN PATENT SPECIFICATION

(11) **EP 1 439 810 B1**
(45) Date of publication and mention of the grant of the patent: **13.07.2011**
(21) Application number: 02773852.5
(22) Date of filing: 22.10.2002
(51) Int. Cl.: A61Q 5/12, A61K 8/41

(54) **ANHYDROUS COSMETIC COMPOSITIONS CONTAINING QUATERNARY AMMONIUM COMPOUNDS**
QUATERNÄRE AMMONIUMVERBINDUNGEN ENTHALTENDE WASSERFREIE ZUSAMMENSETZUNGEN
COMPOSITIONS COSMETIQUES ANHYDRES CONTENANT DES COMPOSES D'AMMONIUM QUATERNAIRE

(30) Priority: 30.10.2001 US 340989 P
(43) Date of publication of application: 28.07.2004
(73) Proprietor: THE PROCTER & GAMBLE COMPANY, Cincinnati, OH 45202 (US)
(72) Inventor: UCHIDA, Mikio, Ashiya-shi, Hyogo 659-0025 (JP); AZUMA, Misa, Yao, Osaka 581-0843 (JP); NAKAJIMA, Kumiko, Kobe, Hyogo 658-0054 (JP); MITSUMATSU, Arata, Hyogo 662-0018 (JP)
(74) Representative: Marollé, Patrick Pierre Pascal
(86) International application number: PCT/US2002/033843
(87) International publication number: WO 2003/037280

(56) References cited:
- EP-A- 0 027 730
- EP-A- 0 636 356
- EP-A- 1 179 339
- EP-A- 1 226 812
- EP-A- 1 250 906
- WO-A-00/38621
- WO-A-02/053113
- US-A- 5 747 109
- "Technical Data Sheet: DEHYQUART(R) L 80" COGNIS WEB PAGE, [Online] XP002228594 Retrieved from the Internet: <URL:http://www.cognis.com/cognis/carechem icals/documents/sheets/dehyquartL80.pdf> [retrieved on 2003-01-23]
- "Technical Data Sheet: DEHYQUART(R) F-75" COGNIS WEB PAGE, [Online] XP002228595 Retrieved from the Internet: <URL:http://www.cognis.com/cognis/carechem icals/documents/sheets/dehyquartF-75.pdf> [retrieved on 2003-01-23]

## Description

### TECHNICAL FIELD

The present invention relates to an anhydrous cosmetic composition containing a quaternary ammonium compound.

### BACKGROUND

A variety of cosmetic products such as hair care products and skin care products have been used to the hair and/or skin. With respect to hair care products, for example, hair shampoo products are used for cleansing the hair by removing excess soil and sebum; hair conditioning products are used for providing various conditioning benefits such as moisturized feel, softness, and static control to the hair; hair styling products are used for setting hair style and/or maintaining hair style; hair color products are used for changing hair color and/or maintaining hair color; and hair growth products are used for encouraging hair growth.

The efficacy of cosmetic products such as hair care products and skin care products are changed by various factors, for example, amount of products applied, how long products are applied on the hair, temperatures of products, the way of applying products to the hair, and so on. Thus, it may not be easy to obtain expected efficacy from cosmetic products such as hair care products and skin care products.

Japanese Patent Lald-open 2001-181156 discloses an anhydrous hair rinse composition comprising polyols, cationic surfactants, C₁₄-C₃₂ fatty alcohols, and silicones, for Improving penetration of treatment components to hair by its warming efficacy. Japanese Patent Laid-open 2007-181156 also discloses that; the cationic surfactants are, for example, stearyl trimethyl ammonium chloride, cetyl trimethyl ammonium chloride, behenyl trimethyl ammonium chloride, and distearyl dimethyl ammonium chloride, etc. .

Patent application WO00/38621 entitled "Self-Warming Hair Conditioning Compositions" also discloses essentially anhydrous hair conditioning compositions. WO00/38621 exemplifies conditioners comprising behentrimonium chloride and sodium potassium alumina silicate. WO00/38621 also discloses a method for conditioning hair with self warming.

It has been found that; it is still not easy to obtain expected conditioning efficacy, especially conditioning benefits such as softness.

One approach to obtain improved conditioning benefits is to increase the level of conditioning agents such as cationic surfactants such as stearyl trimethyl ammonium chloride, cetyl trimethyl ammonium chloride, behenyl trimethyl ammonium chloride, and distearyl dimethyl ammonium chloride, C₁₄-C₃₂ fatty alcohols, and silicones. However, it may not be easy to obtain expected spreadability from cosmetic compositions containing such increased level of conditioning agents. It also may not be easy to manufacture cosmetic compositions containing such increased level of conditioning agents.

Based on the foregoing, there remains a desire for obtaining enhanced efficacy from cosmetic products such as hair care products and skin care products, i.e., there remains a desire for obtaining improved benefits from cosmetic products. There also remains a desire for obtaining improved conditioning benefits such as softness from cosmetic products. There is also a desire for providing cosmetic compositions having improved spreadability on hair and/or skin, and which is easy to manufacture.

None of the existing art provides all of the advantages and benefits of the present invention.

### SUMMARY

The present invention is directed to a cosmetic composition comprising:
(a) a quaternary ammonium compound having at least one group selected from the group consisting of an ester group, an amido group, and mixtures thereof; and
(b) an anhydrous carrier.

The compositions of the present invention can provide improved conditioning benefits such as softness. The compositions of the present invention have improved spreadability on hair and/or skin, and are easy to manufacture.

These and other features, aspects, and advantages of the present invention will become evident to those skilled in the art from a reading of the present disclosure.

### DETAILED DESCRIPTION

While the specification concludes with claims particularly pointing out and distinctly claiming the invention, it is believed the present invention will be better understood from the following description.

All percentages are by weight of the total composition unless otherwise indicated. All ratios are weight ratios unless otherwise indicated. All percentages, ratios, and levels of ingredients referred to herein are based on the actual amount of the ingredient, and do not include solvents, fillers, or other materials with which the ingredient may be combined as commercially available products, unless otherwise indicated.

As used herein, "comprising" means that other steps and other ingredients which do not affect the end result can be added. This term encompasses the terms "consisting of" and "consisting essentially of".

### ANHYDROUS COSMETIC COMPOSITIONS -

Various anhydrous cosmetic compositions such as anhydrous hair care compositions and anhydrous skin care compositions can be used in the present invention. The anhydrous hair care compositions useful herein include, for example, anhydrous hair shampoo compositions, anhydrous hair styling compositions, anhydrous hair conditioning compositions, anhydrous hair color compositions, anhydrous hair growth compositions, and mixtures thereof. The anhydrous skin care compositions useful herein include, for example, anhydrous body shampoo compositions, anhydrous face cleansing compositions, anhydrous skin conditioning compositions, anhydrous shaving compositions, and mixtures thereof. As used in the present invention, "anhydrous" means that the compositions contain 5% or less of water. The anhydrous compositions of the present invention contain, preferably 3% or less of water, more preferably 1% or less of water. Still more preferably, no water is purposely added to the anhydrous composition of the present invention.

The anhydrous cosmetic compositions of the present invention can be in the form of rinse-off products or leave-on products, can be transparent or opaque, and can be formulated in a wide variety of product forms, including but not limited to lotions, creams, gels, emulsions, mousses, and sprays.

The anhydrous cosmetic compositions of the present invention can be mixed with water and applied to the hair and/or skin by any conventional method well known in the art. For example, the anhydrous compositions can be applied to hair and/or skin after mixing with water on hands and/or in a certain vessel. The anhydrous compositions can be applied to wet hair and/or wet skin to mix with water remaining on the hair and/or skin. The anhydrous compositions can be applied to wet and/or dry hair and/or skin to mix with water when rinsed-off.

The anhydrous cosmetic compositions of the present invention can warm by mixing with water. The anhydrous cosmetic compositions of the present invention may warm by a heat from anhydrous carriers when mixing with water, and can also warm by a heat from inorganic heat generating agents when mixing with water if they are included. The anhydrous cosmetic compositions can warm to a temperature of, preferably from about 25°C to about 80°C, more preferably from about 30°C to about 60°C, still more preferably from about 35°C to about 45°C. This temperature can be adjusted by, for example, the amount of the anhydrous carriers when generating a heat. the addition of inorganic heat generating agents, choosing the inorganic heat generating agent when added, the amount of the inorganic heat generating agents when added, and additional agents which can control the heat generating reaction. It is believed that; warming cosmetic compositions such as hair care compositions and skin care compositions can provide enhanced efficacy, i.e., can provide improved benefits. With respect to hair care compositions, for example, it is believed that; warming hair shampoo compositions can provide improved cleansing benefits, warming hair styling compositions can provide improved styling benefits, warming hair conditioning compositions can provide improved hair conditioning benefits due to improved penetration of ingredients, warming hair color compositions and warming hair growth compositions can also provide improved benefits. With respect to skin care compositions, for example, it is believed that; warming body shampoo compositions can provide improved cleansing benefits, warming face cleansing compositions can provide improved cleansing benefits, warming skin conditioning compositions can provide improved conditioning benefits, and warming shaving compositions can provide improved shaving benefits.

### QUATERNARY AMMONIUM COMPOUND

The cosmetic compositions of the present invention comprise quaternary ammonium compounds. Quaternary ammonium compounds useful herein are those having at least one group selected from the group consisting of ester group, amido group, and mixtures thereof. Without intending to be limited by theory, it is also believed that; ester group and/or amido group of the quaternary ammonium compounds useful herein contribute to moisture retention on hair and/or skin, which result in softness on hair and/or skin. It is also believed that; the quaternary ammonium compounds useful herein can provide compositions which have improved spreadability on hair and/or skin and which are easy to manufacture.

The quaternary ammonium compounds useful herein are preferably liquid at 25°C.

The quaternary ammonium compounds useful herein are those having the following formula: 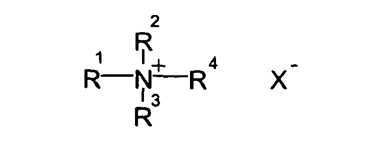 wherein at least one of R¹, R², R³ or R⁴ is independently an ester group or an amido group, the ester group having the following formula:
R⁵-COO-R⁶-
wherein R⁵ is a linear, branched, cyclic, saturated, or unsaturated alkyl group having from 1 to about 30 carbons and being unsubstituted or substituted with a functional group such as hydroxyl group, preferably the alkyl group having from about 8 to about 22 carbons; and R⁶ is a linear, branched, cyclic, saturated, or unsaturated alkyl group having from 1 to about 30 carbons and being unsubstituted or substituted with a functional group such as hydroxyl group, preferably the alkyl group having from about 2 to about 6 carbons, more preferably the alkyl group having from about 2 to about 4 carbons; the amido group having the following formula:
R⁵-CONH-R⁶-
wherein R⁵ and R⁶ are defined as above, for the ester group; the remainder of R¹, R², R³ and R⁴ is independently a linear, branched, cyclic, saturated, or unsaturated alkyl group having 1 to about 30 carbons and being unsubstituted or substituted with a functional group such as hydroxyl group, preferably the alkyl group having from 1 to about 3 carbons, more preferably methyl, ethyl, or hydroxyethyl; and
X is selected from the group consisting of chloride, bromide, iodide, sulfate, hydrosulfate, methylsulfate, ethylsulfate, carbonate, hydrocarbonate, and mixtures thereof, preferably selected from the group consisting of chloride, bromide, iodide, methylsulfate, ethylsulfate, and mixtures thereof.

Preferably, in the quaternary ammonium compounds of the present invention having the above formula, two of R¹, R², R³ or R⁴ are independently the ester group or the amido group, and the remainder of R¹, R², R³ and R⁴ is independently the alkyl group. More preferably, in the quaternary ammonium compounds of the present invention having the above formula, two of R¹, R², R³ or R⁴ are independently the ester group, and the remainder of R¹, R², R³ and R⁴ is independently the alkyl group.

Nonlimiting examples of preferred quaternary ammonium compounds of the present invention include, for example, di-(alkylcarboxymethyl) hydroxyethyl methyl ammonium methosulfate, and methyl bis-(alkylamidoethyl) 2-hydroxyethyl ammonium methosulfate.

Commercially available quaternary ammonium compounds useful herein include: di-(alkylcarboxymethyl) hydroxyethyl methyl ammonium methosulfate with tradenames Rewoquat V3620 and Rewoquat WE15 both available from Goldschmidt, and with tradenames Dehyquart L80 and Dehyquart F75 both available from Cognis; and methyl bis-(alkylamidoethyl) 2-hydroxyethyl ammonium methosulfate with a tradename Varisoft 222 LT-90 available from Goldschmidt.

The quaternary ammonium compounds can be included in the compositions at a level by weight of, preferably from 0.5% to 40%, more preferably from 1% to 20%, still more preferably from 1% to 10%.

### ANHYDROUS CARRIER -

The cosmetic composition of the present invention comprises an anhydrous carrier. The anhydrous carrier can be included in the compositions at a level by weight of, preferably from 10% to 90%, more preferably from 20% to 90%, still more preferably from 25% to 85%.

Anhydrous carrier useful herein includes, for example, liquid polyols such as polyethylene glycol, propylene glycol, butylene glycol, hexylene glycol, glycerin, diglycerin, diethylene glycol, dipropylene glycol, and mixtures thereof; liquid paraffin; mineral oil; vegetable oil; ester oils such as pentaerythritol ester oils, trimethylol ester oils, citrate ester oils, glyceryl ester oils, and mixtures thereof; and mixtures thereof. The liquid polyols such as polyethylene glycol can also be used for generating a heat when mixed with water. The ester oil useful herein is described below as a conditioning agent under the title "Esters". Among them, preferred are liquid polyols such as polyethylene glycol, propylene glycol, butylene glycol, hexylene glycol, glycerin, diglycerin, and mixtures thereof. More preferred is polyethylene glycol in view of its ability to generate a heat by mixing with water and physical properties such as viscosity and fluidity.

The polyethylene glycols useful herein are those having the formula:

H(OCH₂CH₂)ₙ -OH

wherein n has an average value of from 4 to 12. The polyethylene glycol described above is also known as a polyethylene oxide, and polyoxyethylene. Polyethylene glycols useful herein that are especially preferred are PEG-200 wherein n has an average value of about 4. Commercially available preferred polyethylene glycol includes, for example, PEG-200 having trade name Carbowax PEG-200 available from Union Carbide).

### INORCANIC HEAT GENERATING AGENT

The anhydrous cosmetic compositions of the present invention preferably contain an inorganic heat generating agent which generates a heat by mixing with water.

The inorganic heat generating agents useful herein include, for example, chlorides such as calcium chloride (CaCl₂, CaCl₂-H₂O, CaCl₂-2H₂O), magnesium chloride (MgCl₂, MgCl₂-2H₂O, MgCl₂-4H₂O), aluminum chloride (AlCl₃, AlCl₃-6H₂O), ferric chloride (FeCl₃, FeCl₃·2H₂O), and zinc chloride (ZnCl₂); sulfates such as magnesium sulfate (MgSO₄, MgSO₄·H₂O, MgSO₄·4H₂O), zinc sulfate (ZnSO₄·H₂O), ferrous sulfate (FeSO₄, FeSO₄·H₂O), aluminum sulfate (Al(SO₄)₃), calcium sulfate (CaSO₄, CaSO₄·1/2H₂O, CaSO₄·H₂O), and sodium sulfate (Na₂SO₄); dry alum; calcium oxide (CaO); magnesium oxide (MgO); sodium carbonate (Na₂CO₃); zeolite; and sodium hydrogenphosphate (Na₂HPO₄). Preferred are anhydrous inorganic salts such as sodium sulfate (Na₂SO₄), calcium sulfate (CaSO₄), magnesium sulfate (MgSO₄), aluminum sulfate (Al(SO₄)₃), calcium chloride (CaCl₂), magnesium chloride(MgCl₂), calcium oxide (CaO), and mixtures thereof, in view of their effective heat generation, mildness to hair and/or skin, and easy handling. More preferred is anhydrous magnesium sulfate (MgSO₄).

The inorganic heat generating agents useful herein have an average diameter of, preferably from about 0.01 µm to about 40µm, more preferably from about 0.05µm to about 30µm, still more preferably from about 0.1 µm to about 20 µm, in view of preventing gritty feel.

The inorganic heat generating agent can be included in the compositions at a level by weight of, preferably from 5% to 60%, more preferably from 5% to 50%, still more preferably from 10% to 45%.

### POLYOXYALKYLENE DERIVATIVE

The anhydrous cosmetic compositions of the present invention can contain polyoxyalkylene derivatives. The polyoxyalkylene derivatives are preferably contained in the anhydrous cosmetic composition of the present invention when the composition contains the inorganic heat generating agents. It is believed that; polyoxyalkylene derivatives can help the dispersion of inorganic heat generating agents in carriers, thus, prevent the agglomeration of inorganic heat generating agents which causes gritty feel to the skin and /or hair. It is also believed that; some of the polyoxyalkylene derivatives can provide slippery feel which eases gritty feel caused by inorganic heat generating agents.

The polyoxyalkylene derivatives useful herein are preferably water soluble polyoxyalkylene derivatives.

The polyoxyalkylene derivatives useful herein include, for example, polyoxyethylene/polyoxypropylene copolymer, polyoxyethylene alkyl ether, polyoxypropylene alkyl ether, polyoxyethylene alkyl ether ester, polyoxypropylene alkyl ether ester, polyoxyethylene glyceryl ester, polyoxypropylene glyceryl ester, and mixtures thereof. Some of these polyoxyalkylene derivatives can be also used as "NONIONIC SURFACTANT" described below. Among them, polyoxyethylene/polyoxypropylene copolymers are preferably used in view of preventing agglomeration of inorganic heat generating agents, and polyoxyethylene glyceryl esters are preferably used in view of providing slippery feel.

The polyoxyalkylene derivative can be included in the compositions at a level by weight of, preferably from 0.1% to 10%, more preferably from 0.5% to 10%, still more preferably from 1% to 5%.

### Polyoxyethylene/polyoxypropylene copolymer

Preferred polyoxyethylene/polyoxypropylene copolymers included, for example, polyoxyethylene/polyoxypropylene random copolymer and polyoxyethylene/polyoxypropylene block copolymer.

Among these polyoxyalkylene derivatives, polyoxyethytene/polyoxypropylene copolymers including polyoxyethylene/polyoxypropylene random copolymer and polyoxyethylene/polyoxypropylene block copolymer are preferably used in the composition of the present invention in view of their suspending benefit. More preferred is polyoxyethylene/polyoxypropylene block copolymer, still more preferred is polyoxyethylene/polyoxypropylene block copolymer having a weight ratio of polyoxyethylene to polyoxypropylene of from about 5:10 to about 8:10, even more preferred is the block copolymer having the ratio of 8:10.

Commercially available polyoxyalkylene derivatives useful herein include: polyoxyethylene/polyoxypropylene block copolymer, having CTFA name Poloxamer 338, available from BASF under trade name Pluronic F-108, and also available from Sanyo Chemical under trade name Newpol PE-108; and having CTFA name Poloxamer 288, available from BASF under trade name Pluronic F-98, and also available from Sanyo Chemical under trade name Newpol PE-98.

### Polyoxyethylene glyceryl ester

Preferred polyoxyethylene glyceryl esters include, for example, PEG-modified triglycerides with tradenames Tagat TO ®, Tegosoft GC, Tagat BL 276®, Tagat S ®, Tagat S 2 ® (all manufactured by Goldschmidt Chemical Corporation) and with tradenames Crovol A-40, Crovol M-40 (manufactured by Croda Corporation); and PEG-modified glyceryl fatty acid esters such as PEG-20 hydrogenated castor oil, PEG-30 hydrogenated castor oil, PEG-40 hydrogenated castor oil, PEG-45 hydrogenated castor oil, PEG-50 hydrogenated castor oil, PEG-54 hydrogenated castor oil, PEG-55 hydrogenated castor oil, PEG-60 hydrogenated castor oil, PEG-80 hydrogenated castor oil, and PEG-100 hydrogenated castor oil, PEG-30 stearate, PEG-40 stearate, PEG-50 stearate, PEG-75 stearate, PEG-90 stearate, PEG-100 stearate, PEG-120 stearate, and PEG-150 stearate. Among these esters, more preferred are the PEG-modified triglycerides.

### REACTION CONTROL AGENT

The anhydrous cosmetic compositions of the present invention may contain reaction control agents which can control the heat generating reaction of the inorganic heat generating agent. The reaction control agents may slow down the reaction, or accelerate the reaction. The reaction control agents may also control the temperature to which the cosmetic composition warms up.

Acids can be used as reaction control agents for accelerating the reaction of the inorganic heat generating agents. The acid useful herein includes, for example, citric acid, sodium diphosphate, potassium diphophate, ℓ-glutamic acid, lactic acid, hydrochloric acid, malic acid, succinic acid, acetic acid, fumaric acid, ℓ-glutamic acid hydrochloride, tartaric acid, and mixtures thereof; preferably ℓ-glutamic acid, lactic acid, hydrochloric acid, and mixtures thereof. Among the above acids, citric acid is preferably used herein. Some acids can also be used together with amidoamines for providing conditioning benefits as described below. The acid can be contained at a level such that the mole ratio of the inorganic heat generating agent to acid is from 1:0.1 to 1:10, preferably from 1:0.5 to 1:5.

Water absorbing polymer can be used as reaction control agents for slowing down the reaction of the inorganic heat generating agent. The water absorbing polymer useful herein includes, for example, vinyl polymers such as cross linked acrylic acid polymers with the CTFA name Carbomer, carboxylic acid/carboxylate copolymers such as acrylic acid/alkyl acrylate copolymers with the CTFA name Acrylates/C10-30 Alkyl Acrylate Crosspolymer, cellulose derivatives and modified cellulose polymers such as Hydroxyethylcellulose and Hydroxypropyl cellulose, polyvinylpyrrolidone, polyvinyl alcohol, guar gum, other gums, starch-based polymers, alginic acid-based polymers, acrylate polymers, polyalkylene glycols having a molecular weight of more than about 1000, and mixtures thereof. These water absorbing polymers can also be used as the "VISCOSITY MODIFYING AGENT" described below. Among the above water absorbing polymers, preferred are cellulose derivatives and modified cellulose polymers, and more preferred is Hydroxyethylcellulose. The water absorbing polymers can be included in the compositions at a level by weight of, preferably from 0.2% to 20%, more preferably from 0.5% to 15%, still more preferably from 1% to 10%.

### HEAT RESERVING MATERIAL

The anhydrous cosmetic compositions of the present invention may contain heat reserving materials which can reserve a heat. The heat reserving material can be used for prolonging heating, and may be used for slowing down the warming speed, and may also control the temperature to which the cosmetic composition warms up.

The heat reserving materials include, for example, silica gel, carboxymethyl cellulose gel, phase-changing materials, and mixtures thereof. The phase-changing materials useful herein are those which have a melting point of from about 25°C to about 80°C. The phase-changing materials useful herein include, for example, a fatty compound such as fatty alcohol and fatty acid; hydrocarbons; a mixture of hydrocarbons and foamed polyolefin; and mixtures thereof. Fatty compound useful herein are described below under the title "HIGH MELTING POINT FATTY COMPOUND".

The heat reserving material can be included in the compositions at a level by weight of, preferably from 0.2% to 20%, more preferably from 0.5% to 15% still more preferably from 1% to 10%.

### VISCOSITY MODIFYING AGENT

The anhydrous cosmetic composition of the present invention may contain a viscosity modifying agent. The viscosity modifying agent useful herein includes, for example, vinyl polymers such as cross linked acrylic acid polymers with the CTFA name Carbomer, carboxylic acid/carboxylate copolymers such as acrylic acid/alkyl acrylate copolymers with the CTFA name Acrylates/C10-30 Alkyl Acrylate Crosspolymer, cellulose derivatives and modified cellulose polymers, polyvinylpyrrolidone, polyvinyl alcohol, guar gum, other gums, starch-based polymers, alginic acid-based polymers, acrylate polymers, polyalkylene glycols having a molecular weight of more than about 1000, inorganic water soluble material such as bentonite, aluminum magnesium silicate, laponite, hectorite, and anhydrous silicic acid, and mixtures thereof. The polymers described herein can also be used as the "REACTION CONTROL AGENT" described above. Some polyalkylene glycols described herein can also be used as the "ANHYDROUS CARRIER" described above or "POLYPROPYLENE GLYCOL" described below.

The viscosity modifying agent can be included in the compositions at a level by weight of, preferably from 0.01% to 5%, more preferably from 0.05% to 3% still more preferably from 0.1% to 3%.

### HAIR CONDITIONING COMPOSITION

The anhydrous cosmetic compositions of the present invention are preferably anhydrous hair care compositions, more preferably anhydrous hair conditioning compositions. The anhydrous hair conditioning compositions of the present invention preferably comprise additional conditioning agents, for example, high melting point fatty compounds, additional cationic surfactants, cationic polymers, polypropylene glycols, oily conditioning agents such as silicones and ester oils, and mixtures thereof. Among these additional conditioning agents, preferred are high melting point fatty compounds. additional cationic surfactants, and mixtures thereof. The anhydrous hair conditioning compositions of the present invention preferably contain a nonionic surfactant

### HIGH MELTING POINT FATTY COMPOUND

The hair conditioning composition of the present invention preferably contains a high melting point fatty compound. The high melting point fatty compound can be used as the phase changing materials described above under the title "HEAT RESERVING MATERIAL".

The high melting point fatty compound useful herein have a melting point of 25°C or higher, and is selected from the group consisting of fatty alcohols, fatty acids, fatty alcohol derivatives, fatty acid derivatives, and mixtures thereof. Further, it is understood by the artisan that, depending on the number and position of double bonds, and length and position of the branches, certain compounds having certain required carbon atoms may have a melting point of less than 25°C. Such compounds of low melting point are not intended to be included in this section. Nonlimiting examples of the high melting point compounds are found in International Cosmetic Ingredient Dictionary, Fifth Edition, 1993, and CTFA Cosmetic Ingredient Handbook, Second Edition, 1992.

The high melting point fatty compound can be included in the composition at a level by weight of, preferably from 0.1% to 30%, more preferably from 0.2% to 25%, still more preferably from 0.5% to 15%.

The fatty alcohols useful herein are those having from about 14 to about 30 carbon atoms, preferably from about 16 to about 22 carbon atoms. These fatty alcohols are saturated and can be straight or branched chain alcohols. Nonlimiting examples of fatty alcohols include, cetyl alcohol, stearyl alcohol, behenyl alcohol, and mixtures thereof.

The fatty acids useful herein are those having from about 14 to about 30 carbon atoms, preferably from about 12 to about 22 carbon atoms, and more preferably from about 16 to about 22 carbon atoms. These fatty acids are saturated and can be straight or branched chain acids. Also included are diacids, triacids, and other multiple acids which meet the requirements herein. Also included herein are salts of these fatty acids. Nonlimiting examples of fatty acids include lauric add, palmitic acid, stearic acid, behenic acid, sebacic acid, and mixtures thereof.

The fatty alcohol derivatives and fatty acid derivatives useful herein include alkyl ethers of fatty alcohols, alkoxylated fatty alcohols, alkyl ethers of alkoxylated fatty alcohols, esters of fatty alcohols, fatty acid esters of compounds having esterifiable hydroxy groups, hydroxy-substituted fatty acids, and mixtures thereof. Nonlimiting examples of fatty alcohol derivatives and fatty acid derivatives include materials such as methyl stearyl ether, the ceteth series of compounds such as ceteth-1 through ceteth-45, which are ethylene glycol ethers of cetyl alcohol, wherein the numeric designation indicates the number of ethylene glycol moieties present; the steareth series of compounds such as steareth-1 through 10, which are ethylene glycol ethers of steareth alcohol, wherein the numeric designation indicates the number of ethylene glycol moieties present; ceteareth 1 through ceteareth-10, which are the ethylene glycol ethers of ceteareth alcohol, i.e. a mixture of fatty alcohols containing predominantly cetyl and stearyl alcohol, wherein the numeric designation indicates the number of ethylene glycol moieties present; C₁-C₃₀ alkyl ethers of the ceteth, steareth, and ceteareth compounds just described; polyoxyethylene ethers of behenyl alcohol; ethyl stearate, cetyl stearate, cetyl palmitate, stearyl stearate, myristyl myristate, polyoxyethylene cetyl ether stearate, polyoxyethylene stearyl ether stearate, polyoxyethylene lauryl ether stearate, ethylene glycol monostearate, polyoxyethylene monostearate, polyoxyethylene distearate, propyleneglycol monostearate, propyleneglycol distearate, trimethylolpropane distearate, sorbitan stearate, polyglyceryl stearate, glyceryl monostearate, glyceryl distearate, glyceryl tristearate, and mixtures thereof.

Commercially available high melting point fatty compounds useful herein include: cetyl alcohol, stearyl alcohol, and behenyl alcohol having tradenames KONOL series available from Shin Nihon Rika (Osaka, Japan), and NAA series available from NOF (Tokyo, Japan); pure behenyl alcohol having tradename 1-DOCOSANOL available from WAKO (Osaka, Japan), various fatty acids having tradenames NEO-FAT available from Akzo (Chicago Illinois, USA), HYSTRENE available from Witco Corp. (Dublin Ohio, USA), and DERMA available from Vevy (Genova, Italy).

### ADDITIONAL CATIONIC SURFACTANT

The hair conditioning composition of the present invention preferably contains additional cationic surfactants. The additional cationic surfactant can be included in the composition at a level by weight of, preferably from 0.1% to 10%, more preferably from 0.25% to 8%, still more preferably from 0.5% to 5%,

Nonlimiting examples of preferred additional cationic surfactants include: behenyl trimethyl ammonium chloride available, for example, with tradename INCROQUAT TMC-80 from Croda and ECONOL TM22 from Sanyo Kasei, distearyl dimethyl ammonium chloride available, for example, with tradename Varisoft TA 100 from Goldschmidt, cetyl trimethyl ammonium chloride available, for example, with tradename CA-2350 from Nikko Chemicals, hydrogenated tallow alkyl trimethyl ammonium chloride, dialkyl (14-18) dimethyl ammonium chloride, ditallow alkyl dimethyl ammonium chloride, dihydrogenated tallow alkyl dimethyl ammonium chloride, dicetyl dimethyl ammonium chloride, di(behenyl/arachidyl) dimethyl ammonium chloride, dibehenyl dimethyl ammonium chloride, stearyl dimethyl benzyl ammonium chloride, stearyl propyleneglycol phosphate dimethyl ammonium chloride, stearoyl amidopropyl dimethyl benzyl ammonium chloride, stearoyl amidopropyl dimethyl (myristylacetate) ammonium chloride, and N-(stearoyl colamino formyl methy) pyridinium chloride.

Salts of amidoamines and acids can be used as additional cationic surfactants herein. The amidoamine useful herein are those having the following general formula:

R¹ CONH (CH₂)ₘ N (R²)₂

wherein R¹ is a residue of C₁₁ to C₂₄ fatty acids, R² is a C₁ to C₄ alkyl, and m is an integer from 1 to 4. Preferred amidoamines useful in the present invention includes stearamidopropyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylamine, behenamidoethyldiethylamine, behenamidoethyldimethylamine, arachidamidopropyldimethylamine, arachidamidopropyldiethylamine, arachidamidoethyldiethylamine, arachidamidoethyldimethylamine, and mixtures thereof; more preferably stearamidopropyldimethylamine, stearamidoethyldiethylamine, and mixtures thereof. Commercially available amidoamines useful herein include: stearamidopropyldimethylamine having tradename SAPDMA available from Inolex, and tradename Amidoamine MPS available from Nikko. The acids useful herein are selected from the group consisting of ℓ-glutamic acid, lactic acid, hydrochloric acid, malic acid, succinic acid, acetic acid, fumaric acid, ℓ-glutamic acid hydrochloride, tartaric acid, and mixtures thereof; preferably ℓ-glutamic acid, lactic acid, hydrochloric acid, and mixtures thereof. The acid described herein can also be used as the "REACTION CONTROL AGENT" described above. The acid can be contained at a level such that the mole ratio of amidoamine to acid is, preferably from about 1:0.3 to about 1:1, more preferably from about 1:0.5 to about 1:0.9. Commercially available acids useful herein include: ℓ-Glutamic acid: ℓ-Glutamic acid (cosmetic grade) available from Ajinomoto.

### POLYPROPYLENE GLYCOL

The anhydrous hair care composition of the present invention may contain polypropylene glycols.

Polypropylene glycols useful herein are those having a molecular weight of from about 200g/mol to about 100,000g/mol, preferably from about 500g/mol to about 60,000g/mol, more preferably from about 1,000g/mol to about 10,000g/mol, in view of having certain required water solubility and compatibility with anhydrous carriers. Some polypropylene glycols described herein can also be used as the "VISCOSITY MODIFYING AGENT" described above.

The polypropylene glycol can be included in the composition at a level by weight of, preferably from 2% to 60%, more preferably from 5% to 50%, still preferably from 10% to 40% in view of providing conditioning benefits such as moisturizing benefit while providing reduced stickiness.

Preferably the polypropylene glycol is selected from the group consisting of a single-polypropylene glycol-chain segment polymer, a multi-polypropylene glycol-chain segment polymer, and mixtures thereof, more preferably selected from the group consisting of a single-polypropylene glycol-chain segment polymer of Formula I, below. HO-(C₃H₆O)ₐH(I) wherein a is a value from about 4 to about 400, preferably from about 10 to about 100, and more preferably from about 20 to about 40.

The single-polypropylene glycol-chain segment polymer useful herein is typically inexpensive, and is readily available from, for example, Sanyo Kasei (Osaka, Japan), Dow Chemicals (Midland, Michigan, USA), Calgon Chemical, Inc. (Skokie, Illinois, USA). Arco Chemical Co. (Newton Square Pennsylvania, USA), Witco Chemicals Corp. (Greenwich, Connecticut, USA), and PPG Specialty Chemicals (Gumee, Illinois, USA).

In a preferred embodiment, one or more of the propylene oxide repeating groups in the polypropylene glycol is an isopropyl oxide repeating group. More preferably, substantially all of the propylene oxide repeating groups of the polypropylene glycol are isopropyl oxide repeating groups. Accordingly, a highly preferred single-polypropylene glycol-chain segment polymer has the formula: 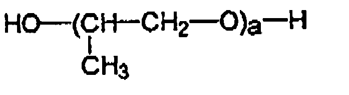 wherein a is defined as described above for Formula I. It is recognized that the isopropyl oxide repeating groups may also correspond either alone, or in combination with the above depicted, to:

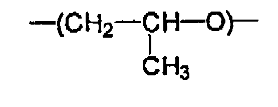

The highly preferred single-polypropylene glycol-chain segment polymer is readily available from, for example, Sanyo Kasei (Osaka, Japan) as New pol PP-2000 and New pol PP-4000.

The multi-polypropylene glycol-chain segment polymer is available from, for example. Sanyo Kasei as New Pol GP-4000 and New Pol SP-4000, Croda, Inc. as Probutyl DB-10.

### OILY CONDITIONING AGENT

The anhydrous cosmetic compositions of the present invention may contain oily conditioning agents. The oily conditioning agents can be included in the composition at a level by weight of, preferably from 0,1% to 20%, more preferably from 1% to 10%, still preferably from 2% to 10% in view of providing conditioning benefits such as softness and smoothness.

The oily conditioning agents useful herein include, for example, paraffins, esters, silicones, fatty compounds, mineral oils, hydrocarbons, poly α-olefin oils, vegetable oils, and mixtures thereof. Among these oily conditioning agents, preferred are liquid oily conditioning agents and selected from the group consisting of paraffins, esters, silicones, and mixtures thereof. More preferred are silicone oils in view of providing conditioning benefits such as softness and smoothness.

### Ester

Esters preferably used herein are those having a melting point of less than 25°C. Such esters include, for example, pentaerythritol ester oils, trimethylol ester oils, citrate ester oils, glyceryl ester oils, and mixtures thereof. Particularly useful pentaerythritol ester oils and trimethylol ester oils herein include pentaerythritol tetraisostearate, pentaerythritol tetraoleate, trimethytolpropane triisostearate, trimethylolpropane trioleate, and mixtures thereof. Such compounds are available from Kokyo Alcohol with tradenames KAKPTI, KAKTTI, and Shin-nihon Rika with tradenames PTO, ENUJERUBU TP3SO. Particularly useful citrate ester oils herein include triisocetyl citrate with tradename CITMOL 316 available from Bernel, triisostearyl citrate with tradename PELEMOL TISC available from Phoenix, and trioctytdodecyl citrate with tradename CITMOL 320 available from Bemel. Particularly useful glyceryl ester oils herein include triisostearin with tradename SUN ESPOl. G-318 available from Taiyo Kagaku, triolein with tradename CITHROL GTO available from Croda Surfactants Ltd., trilinolein with tradename EFADERMA-F available from Vevy, or tradename EFA-GLYCERIDES from Brooks. The ester oils described herein can also be used as the "ANHYDROUS CARRIER" described above.

### Silicone

The silicone hereof can include volatile or nonvolatile silicone conditioning agents.

The silicones for use herein preferably have a viscosity of from about 5 to about 2,000,000 centistokes at 25°C. more preferably from about 500 to about 1,800,000, and even more preferably from about 5,000 to about 1,500,000. The viscosity can be measured by means of a glass capillary viscometer as set forth in Dow Corning Corporate Test Method CTM0004, July 20, 1970 .

Preferred silicone compounds are polydimethylsiloxane, polydiethylsiloxane, and polymethylphenylsiloxane. Polydimethylsiloxane, which is also known as dimethicone, is especially preferred. The polyalkylsiloxanes that can be used include, for example, polydimethylsiloxanes. These silicone compounds are available, for example, from GE Toshiba Silicones, from the General Electric Company in their ViscasilR and SF 96 series, and from Dow Corning in their Dow Corning 200 series. Dimethicones having a viscosity of about 10,000 centistokes, for example, from GE Toshiba Silicones with a tradename TSF451-1MA are highly preferred herein.

Another preferred silicone compound is a silicone gum. The term "silicone gum", as used herein, means a polyorganosiloxane material having a viscosity at 25°C of greater than or equal to 1,000,000 centistokes. It is recognized that the silicone gums described herein can also have some overlap with the above-disclosed silicone compounds. This overlap is not intended as a limitation on any of these materials. Silicone gums are described by Petrarch, and others including U.S. Patent No. 4,152,416, to Spitzer et al., issued May 1, 1979 and Noll, Walter, Chemistry and Technology of Silicones, New York: Academic Press 1968. Also describing silicone gums are General Electric Silicone Rubber Product Data Sheets SE 30, SE 33, SE 54 and SE 76.

The "silicone gums" will typically have a mass molecular weight in excess of about 200,000, generally between about 200,000 and about 1,000,000. Specific examples include polydimethylsiloxane, poly(dimethylsiloxane methylvinylsiloxane) copolymer, poly(dimethylsiloxane diphenylsiloxane methylvinylsiloxane) copolymer and mixtures thereof. These silicone gums are preferably used in the form of mixtures with solvents such as cyclomethicone. Such mixtures have a viscosity of preferably from about 5,000 to about 100,000 centistokes.

Polyalkylerieoxide-modified siloxanes useful herein include, for example, polypropylene oxide modified and polyethylene oxide modified polydimethylsiloxane. The ethylene oxide and polypropylene oxide level should be sufficiently low so as not to interfere with the dispersibility characteristics of the silicone. These materials are also known as dimethicone copolyols.

Amino-substituted siloxanes known as "amodimethicone" are also useful herein. Especially preferred amino-substituted siloxane is a polymer known as "trimethylsilylamodimethicone". Another preferred amino-substituted siloxanes are those having the tradename "UCAR SILICONE ALE 56" available from Union Carbide.

Also useful are silicone resins, which are highly crosslinked polymeric siloxane systems. Preferred resins are offered by General Electric as GE SS4230 and SS4267. Silicone resins in particular, can conveniently be identified according to a shorthand nomenclature system well known to those skilled in the art as the "MDTQ" nomenclature. Under this system, the silicone is described according to the presence of various siloxane monomer units which make up the silicone. Briefly, the symbol M denotes the mono-functional unit (CH₃)₃SiO_{0,5}; D denotes the difunctional unit (CH₃)₂SiO; T denotes the trifunctional unit (CH₃)SiO₁₅; and Q denotes the quadri- or tetra-functional unit SiO2. The silicone resins for use herein which are preferred are MQ, MT, MTQ, MQ and MDTQ resins. Especially preferred are MQ resins wherein the M:Q ratio is from 0.5:1.0 to 1.5:1.0 and the average molecular weight of the resin is from about 1000 to about 10,000.

### NONIONIC SURFACTANT

The anhydrous hair care composition of the present invention preferably contains nonionic surfactant in view of providing a physical stability. The nonionic surfactant can be included in the composition of the present invention at a level by weight of, preferably from 0.01% to 10%, more preferably from 0.05% to 8%, still preferably from 0.1 % to 5%.

The nonionic surfactant useful herein includes, for example, polyoxyethylene glyceryl esters such as PEG-modified triglycerides with tradenames Tagat TO ® available from Goldschmidt Chemical Corporation, PEG-60 hydrogenated castor oil, and PEG-100 stearate; ethylene glycol ethers of fatty alcohols such as ceteareth-20; alkyl polysaccharide surfactants such as alkyl polyglycosides; long chain tertiary amine oxides such as lauramine oxide; and long chain tertiary phosphine oxides such as lauryl dimethyl phosphine oxide. Polyoxyethylene glyceryl esters useful herein are described above under the title "POLYOXYALKYLENE DERIVATIVE". Among them, preferred are polyoxyethylene glyceryl esters and ethylene glycol ethers of fatty alcohols.

### ADDITIONAL COMPONENTS

The hair conditioning composition of the present invention may include other additional components, which may be selected by the artisan according to the desired characteristics of the final product and which are suitable for rendering the composition more cosmetically or aesthetically acceptable or to provide them with additional usage benefits.

A wide variety of other additional components can be formulated into the present compositions. These include: other conditioning agents such as hydrolyzed collagen with tradename Peptein 2000 available from Hormel, vitamin E with tradename Emix-d available from Eisai, panthenol available from Roche, panthenyl ethyl ether available from Roche, a mixture of Polysorbate 60 and Cetearyl Alcohol with tradename Polawax NF available from Croda Chemicals, glycerylmonostearate available from Stepan Chemicals, hydroxyethyl cellulose available from Aqualon, 3-pyridinecarboxy acid amide (niacinamide), hydrolysed keratin, proteins, plant extracts, and nutrients; hair-fixative polymers such as amphoteric fixative polymers, cationic fixative polymers, anionic fixative polymers, nonionic fixative polymers, and silicone grafted copolymers; preservatives such as benzyl alcohol, methyl paraben, propyl paraben and imidazolidinyl urea; pH adjusting agents, such as citric acid, sodium citrate, succinic acid, phosphoric acid, sodium hydroxide, sodium carbonate; salts, in general, such as potassium acetate and sodium chloride; coloring agents, such as any of the FD&C or D&C dyes; hair oxidizing (bleaching) agents, such as hydrogen peroxide, perborate and persulfate salts; hair reducing agents such as the thioglycolates; perfumes; and sequestering agents, such as disodium ethylenediamine tetra-acetate; ultraviolet and infrared screening and absorbing agents such as octyl salicylate, antidandruff agents such as zinc pyridinethione, and salicylic acid; and optical brighteners, for example polystyrylstilbenes, triazinstilbenes, hydroxycoumarins, aminocoumarins, triazoles, pyrazolines, oxazoles, pyrenes, porphyrins, imidazoles, and mixtures thereof; non-heat generating particles such as cellulose particles, mica, silica, mud, clay, and mixtures thereof.

Other additional components generally are used individually at levels of from 0.001% to 10%, preferably up to 5% by weight of the composition.

### EXAMPLES

The following examples further describe and demonstrate embodiments within the scope of the present invention. Ingredients are identified by Chemical or CTFA name, or otherwise defined below. All percentages herein are based upon the total weight of the compositions, and all such weight percentages as they pertain to listed ingredients are based on the active level and, therefore, do not include carriers or by-products that may be included in commercially available materials.

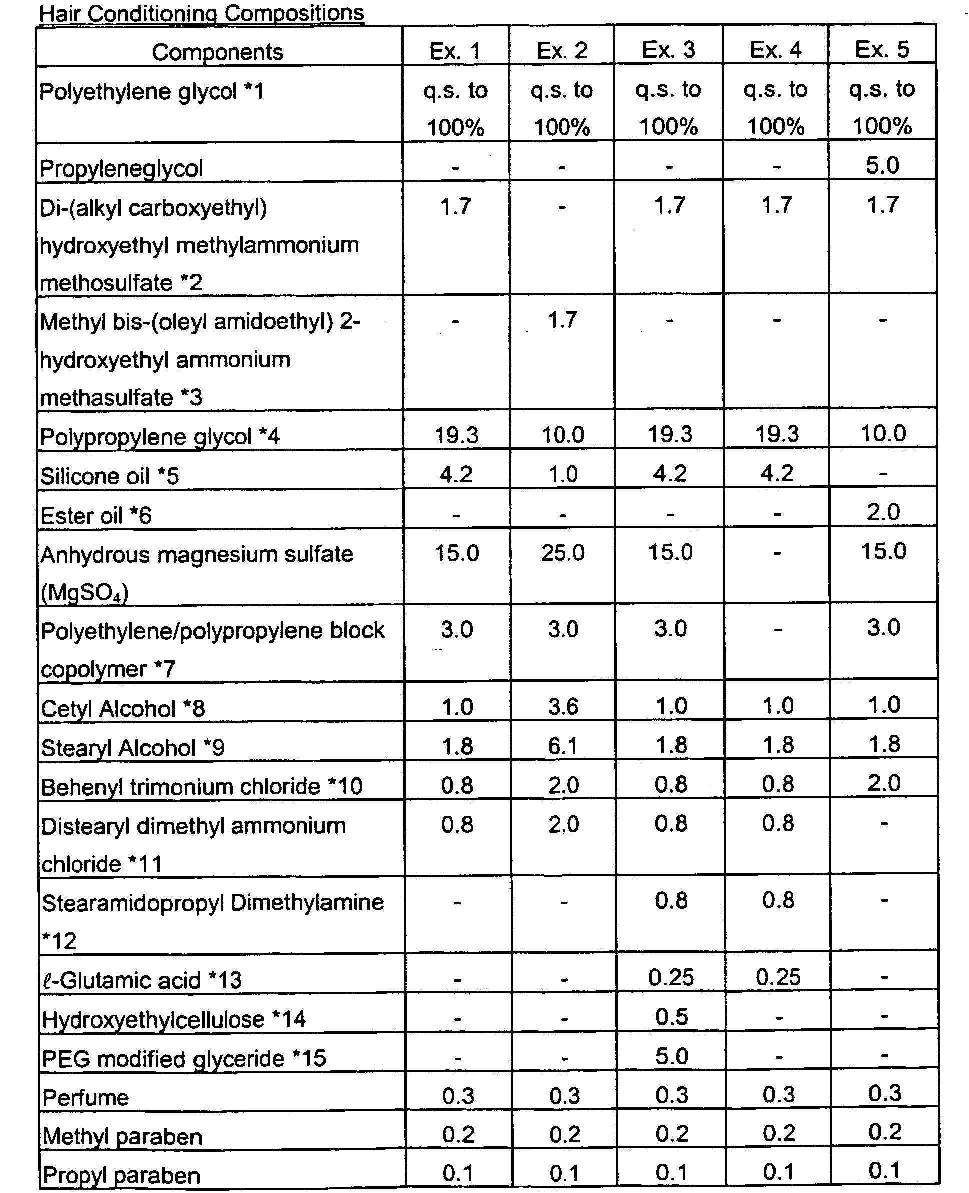

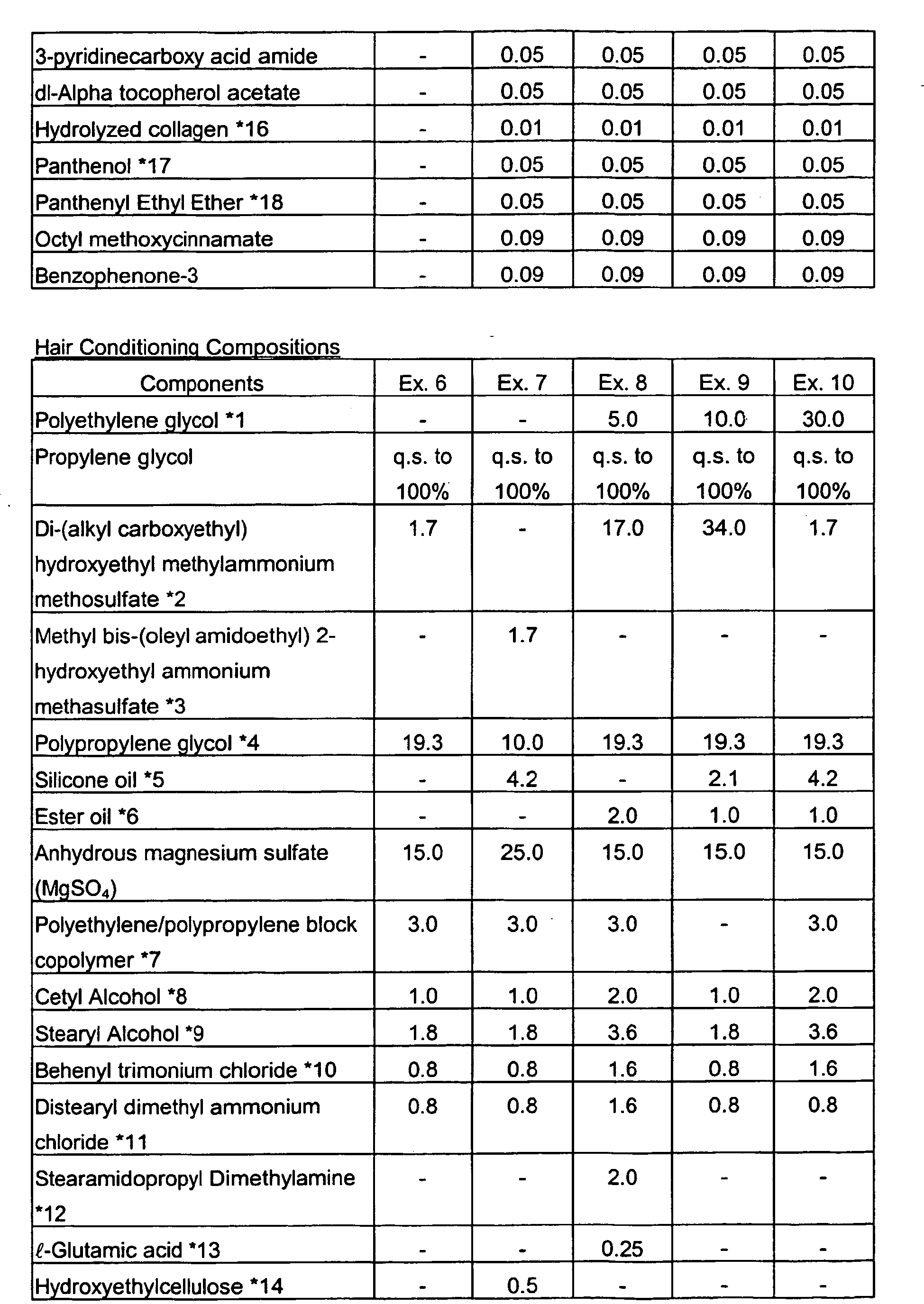

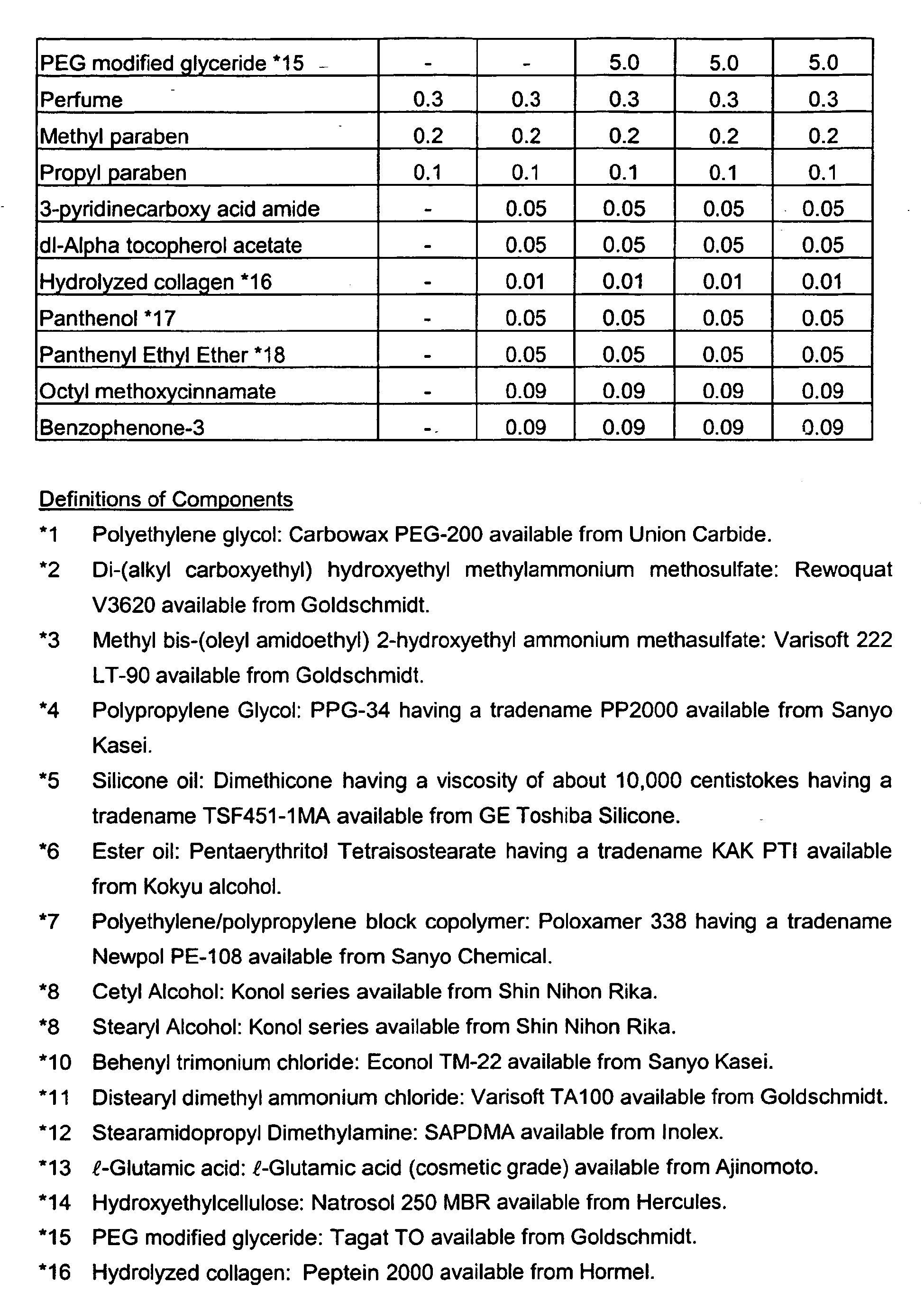

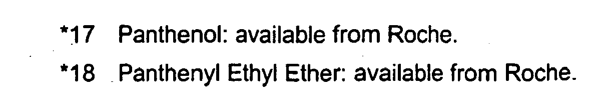

### Method of Preparation

The hair conditioning compositions of Examples 1 through 10 as shown above can be prepared by any conventional method well known in the art. They are suitably made as follows: Quaternary ammonium compounds having ester groups and/or amido groups, and when included in the composition, polypropylene glycol, PEG modified glyceride, and other carriers are added to anhydrous carrier to make a mixture. When included in the composition, polymeric materials such as hydroxyethylcellulose can be dispersed in the mixture at room temperature to make a polymer solution, and heated up to above 70°C. When included in the composition, polyoxyalkylene derivatives such as polyoxyethylene/polyoxyalkylene copolymer, amidoamines and acids, cationic surfactants, high melting point fatty compounds, and ester oils are added in the solution with agitation. Then, when included in the composition, inorganic heat generating agents such as magnesium sulfate are also added in the solution with agitation. The mixture thus obtained is cooled down to about 30°C, and the remaining components such as silicone compound are added with agitation.

### Method of Use

The hair conditioning compositions of Examples 1 through 10 as shown above can be mixed with water and applied to the hair and/or skin by any conventional method well known in the art. For example, the anhydrous compositions can be applied to hair and/or skin after mixing with water on hands and/or in a certain vessel. The anhydrous compositions can be applied to wet hair and/or wet skin to mix with water remaining on the hair and/or skin. The anhydrous compositions can be applied to wet and/or dry hair and/or skin to mix with water when rinsed-off. The hair conditioning compositions of Examples 1 through 10 as shown above are preferably applied to wet hair to mix with water remaining on the hair.

The embodiments disclosed herein have many advantages. For example, the hair conditioning compositions of Examples 1 through 10 as shown above provide improved conditioning benefits such as softness. The hair conditioning compositions of Examples 1 through 10 as shown above have improved spreadability on hair and/or skin, and are easy to manufacture. The hair conditioning compositions of Examples 1 through 10 as shown above warm to a temperature such that the user can perceive a warm feeling, when mixing with water. For example, the hair conditioning composition of Example 1 as shown above can warm up to a temperature of from about 30°C to about 50°C, when applied to wet hair and mixed with water remaining on the hair.

## Claims

1. An anhydrous cosmetic composition comprising:
(a) a quaternary ammonium compound having at least one ester group and being liquid at 25°C,
(b) an anhydrous carrier
(c) 5% to 60% of an inorganic heat-generating agent which generates heat by mixing with water selected from an anhydrous inorganic salt and
(d) 5% or less of water.

2. An anhydrous compositions according to claim 1 wherein said quaternary ammonium compound has the formula: 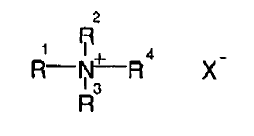 wherein two of R¹, R², R³ and R⁴ are independently an ester group having the following formula:
R⁵-COO-R⁶-
wherein R⁵ is a linear, branched, cyclic, saturated, or unsaturated alkyl group having from 1 to 30 carbons and being unsubstituted or substituted with a functional group; and R⁶ is a linear, branched, cyclic, saturated, or unsaturated alkyl group having from 1, to 30 carbons and being unsubstituted or
substituted with a functional group;
the remainder of R¹, R², R³ and R⁴ is independently a linear, branched, cyclic, saturated, or unsaturated alkyl group having 1 to 30 carbons and being unsubstituted or substituted with a hydroxyl group; and
X is selected from the group consisting of chloride, bromide, iodide, sulfate, hydrosulfate, methylsulfate, ethylsulfate, carbonate, hydrocarbonate, and mixtures thereof: and
wherein the quaternary ammonium compound is liquid at 25°C; and wherein
(b) said anhydrous carrier, is selected from the group consisting of propylene glycol, butylene glycol, hexylene glycol, glycerin, diglycerin, polyethylene glycol, and mixtures thereof.

3. The cosmetic composition according to Claim 1 wherein said inorganic heat generating agent which generates a heat hy mixing with water, is elected from the group consisting of sodium sulfate, calcium sulfate, magnesium sulfate, aluminum sulfate, calcium chloride, magnesium chloride, calcium oxide, and mixtures thereof.

4. The cosmetic composition according to Claim 3 further comprising a polyoxyalkylene derivative, preferably the polyoxyalkylene derivative selected from the group consisting of polyoxyethylene/polyoxypropylene copolymer, polyoxyethylene alkyl ether, polyoxypropylene alkyl ether, polyoxyethylene alkyl ether ester, polyoxypropylene alkyl ether ester, polyoxyethylene glyceryl ester, polyoxypropylene glyceryl ester, and mixtures thereof.#

5. The cosmetic composition according to Claim 1, wherein said anhydrous composition further comprises an additional cationic surfactant.

6. The cosmetic composition according to Claim 1, wherein said anhydrous composition further comprises a fatty compound having a melting point of 25°C or more.

7. The cosmetic composition according Lo Claim 1, said anhydrous composition comprises by weight:
(a) from 0.5% to 40% of said quaternary ammonium compound;
(b) from 10% to 90% of said anhydrous carrier;
(c) from, 0.1% to 30% of said fatty compound having a melting point of 25°C or more;
(d) from 0.1% to 10% of an additional cationic surfactant;
(c) from 5% to 60% of an inorganic heat generating agent which generates a heat by mixing with water; and
(f) from 0.1% to 10% of a polyoxyalkylene derivative.

8. A method of conditioning hair comprising following steps: applying the hair conditioning composition according to Claim 1. to wet hair; and mixing the hair conditioning composition with water remaining on the hair

9. The use of an anhydrous cosmetic composition according to claim 1 as a hair care composition.

## Patentansprüche

1. Nichtwässrige Kosmetikzusammensetzung, umfassend:
(a) eine quartäre Ammoniumverbindung, die mindestens eine Estergruppe aufweist und bei 25 °C flüssig ist,
(b) einen wasserfreien Träger
(c) zu 5 % bis 60 % ein anorganisches Wärmeerzeugungsmittel, das durch Vermischen mit Wasser Wärme erzeugt und das aus einem wasserfreien anorganischen Salz ausgewählt ist, und
(d) zu 5 % oder weniger Wasser.

2. Wasserfreie Zusammensetzung nach Anspruch 1, wobei die quartäre Ammoniumverbindung die folgende Formel aufweist: 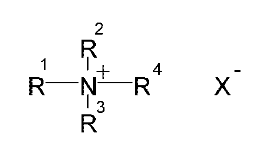 wobei zwei von R¹, R², R³ und R⁴ unabhängig voneinander eine Estergruppe mit der folgenden Formel sind:
R⁵-COO-R⁶⁻
wobei R⁵ eine lineare, verzweigte, cyclische, gesättigte oder ungesättigte Alkylgruppe mit 1 bis 30 Kohlenstoffen ist und unsubstituiert oder mit einer funktionellen Gruppe substituiert ist; und R⁶ eine lineare, verzweigte, cyclische, gesättigte oder ungesättigte Alkylgruppe mit 1 bis 30 Kohlenstoffen ist und unsubstituiert oder mit einer funktionellen Gruppe substituiert ist;
die übrigen von R¹, R², R³ und R⁴ unabhängig voneinander eine lineare, verzweigte, cyclische, gesättigte oder ungesättigte Alkylgruppe mit 1 bis 30 Kohlenstoffen sind und unsubstituiert oder mit einer Hydroxylgruppe substituiert sind; und
X ausgewählt ist aus der Gruppe bestehend aus Chlorid, Bromid, Iodid, Sulfat, Hydrogensulfat, Methylsulfat, Ethylsulfat, Carbonat, Hydrogencarbonat und Mischungen davon; und
wobei die quartäre Ammoniumverbindung bei 25 °C flüssig ist; und wobei (b) der wasserfreie Träger ausgewählt ist aus der Gruppe bestehend aus Propylenglycol, Butylenglycol, Hexylenglycol, Glycerin, Diglycerin, Polyethylenglycol und Mischungen davon.

3. Kosmetikzusammensetzung nach Anspruch 1, wobei das anorganische Wärmeerzeugungsmittel, das durch Vermischen mit Wasser Wärme erzeugt, ausgewählt ist aus der Gruppe bestehend aus Natriumsulfat, Calciumsulfat, Magnesiumsulfat, Aluminiumsulfat, Calciumchlorid, Magnesiumchlorid, Calciumoxid und Mischungen davon.

4. Kosmetikzusammensetzung nach Anspruch 3, ferner umfassend ein Polyoxyalkylenderivat, wobei das Polyoxyalkylenderivat vorzugsweise ausgewählt ist aus der Gruppe bestehend aus Polyoxyethylen/Polyoxypropylen-Copolymer, Polyoxyethylenalkylether, Polyoxypropylenalkylether, Polyoxyethylenalkyletherester, Polyoxypropylenalkyletherester, Polyoxyethylenglycerylester, Polyoxypropylenglycerylester und Mischungen davon.

5. Kosmetikzusammensetzung nach Anspruch 1, wobei die wasserfreie Zusammensetzung ferner ein zusätzliches kationisches Tensid umfasst.

6. Kosmetikzusammensetzung nach Anspruch 1, wobei die wasserfreie Zusammensetzung ferner eine Fettverbindung mit einem Schmelzpunkt von 25 °C oder mehr umfasst.

7. Kosmetikzusammensetzung nach Anspruch 1, wobei die wasserfreie Zusammensetzung bezogen auf das Gewicht Folgendes umfasst:
(a) von 0,5 % bis 40 % die quartäre Ammoniumverbindung;
(b) von 10 % bis 90 % den wasserfreien Träger;
(c) von 0,1 % bis 30 % die Fettverbindung mit einem Schmelzpunkt von 25 °C oder mehr;
(d) von 0,1 % bis 10 % ein zusätzliches kationisches Tensid;
(e) von 5 % bis 60 % ein anorganisches Wärmeerzeugungsmittel, das durch Vermischen mit Wasser Wärme erzeugt; und
(f) von 0,1 % bis 10 % ein Polyoxyalkylenderivat.

8. Verfahren zum Konditionieren von Haar, das die folgenden Schritte umfasst: Auftragen der Haarkonditionierungszusammensetzung nach Anspruch 1 auf nasses Haar; und Mischen der Haarkonditionierungszusammensetzung mit auf dem Haar verbleibendem Wasser.

9. Verwendung einer wasserfreien Kosmetikzusammensetzung nach Anspruch 1 als Haarpflegezusammensetzung.

## Revendications

1. Composition cosmétique anhydre comprenant :
(a) un composé d'ammonium quaternaire ayant au moins un groupe ester et étant liquide à 25 °C,
(b) un véhicule anhydre
(c) 5 % à 60 % d'un agent générant de la chaleur inorganique qui génère de la chaleur par mélange avec de l'eau choisi parmi un sel inorganique anhydre et
(d) 5 % ou moins d'eau.

2. Composition anhydre selon la revendication 1, dans laquelle ledit composé d'ammonium quaternaire est de formule : 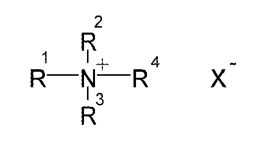 dans laquelle deux parmi R¹, R², R³ et R⁴ sont indépendamment un groupe ester ayant la formule suivante :
R⁵-COO-R⁶⁻
dans laquelle R⁵ est un groupe alkyle linéaire, ramifié, cyclique, saturé ou insaturé comportant de 1 à 30 carbones et étant non substitué ou substitué avec un groupe fonctionnel ; et R⁶ est un groupe alkyle linéaire, ramifié, cyclique, saturé ou insaturé comportant de 1 à 30 carbones et étant non substitué ou substitué avec un groupe fonctionnel ;
le reste de R¹, R², R³ et R⁴ est indépendamment un groupe alkyle linéaire, ramifié, cyclique, saturé ou insaturé comportant 1 à 30 carbones et étant non substitué ou
substitué avec un groupe hydroxyle ; et
X est choisi dans le groupe constitué de chlorure, bromure, iodure, sulfate, hydrosulfate, méthylsulfate, éthylsulfate, carbonate, hydrocarbonate, et leurs mélanges ; et
dans laquelle le composé d'ammonium quaternaire est liquide à 25 °C ; et dans laquelle (b) ledit véhicule anhydre est choisi dans le groupe constitué de propylène glycol, butylène glycol, hexylène glycol, glycérine, diglycérine, polyéthylène glycol, et leurs mélanges.

3. Composition cosmétique selon la revendication 1, dans laquelle l'agent de génération de chaleur inorganique qui génère une chaleur par mélange avec de l'eau, est choisi dans le groupe constitué de sulfate de sodium, sulfate de calcium, sulfate de magnésium, sulfate d'aluminium, chlorure de calcium, chlorure de magnésium, oxyde de calcium, et leurs mélanges.

4. Composition cosmétique selon la revendication 3, comprenant en outre un dérivé de polyoxyalkylène, de préférence le dérivé de polyoxyalkylène étant choisi dans le groupe constitué d'un copolymère polyoxyéthylène/polyoxypropylène, un polyoxyéthylène alkyléther, un polyoxypropylène alkyléther, un ester de polyoxyéthylène alkyléther, un ester de polyoxypropylène alkyléther, un ester de polyoxyéthylène glycéryle, un ester de polyoxypropylène glycéryle, et leurs mélanges.#

5. Composition cosmétique selon la revendication 1, où ladite composition anhydre comprend en outre un agent tensioactif cationique supplémentaire.

6. Composition cosmétique selon la revendication 1, où ladite composition anhydre comprend en outre un composé gras ayant un point de fusion de 25 °C ou plus.

7. Composition cosmétique selon la revendication 1, ladite composition anhydre comprend en poids :
(a) de 0,5 % à 40 % dudit composé d'ammonium quaternaire ;
(b) de 10 % à 90 % dudit véhicule anhydre ;
(c) de 0,1 % à 30 % dudit composé gras ayant un point de fusion de 25 °C ou plus ;
(d) de 0,1 % à 10 % d'un agent tensioactif cationique supplémentaire ;
(e) de 5 % à 60 % d'un agent de génération de chaleur inorganique qui génère une chaleur par mélange avec de l'eau ; et
(f) de 0,1 % à 10 % d'un dérivé de polyoxyalkylène.

8. Procédé de conditionnement des cheveux comprenant les étapes suivantes :
application de la composition de conditionnement des cheveux selon la revendication 1 sur des cheveux mouillés ; et mélange de la composition de conditionnement des cheveux avec l'eau restant sur les cheveux.

9. Utilisation d'une composition cosmétique anhydre selon la revendication 1 en tant que composition pour soins capillaires.
